# EUROPÄISCHE PATENTSCHRIFT

(11) **EP 3 021 751 B1**
(45) Veröffentlichungstag und Bekanntmachung des Hinweises auf die Patenterteilung: **25.10.2017**
(21) Anmeldenummer: 14777266.9
(22) Anmeldetag: 26.09.2014
(51) Int. Cl.: A61K 45/06, A61K 31/445, A61K 31/727, A61B 5/15, A61B 5/151, A61B 5/157, A61M 37/00

(54) **BLUTENTNAHMEVORRICHTUNG**
BLOOD WITHDRAWAL DEVICE
DISPOSITIF DE PRÉLÈVEMENT SANGUIN

(30) Priorität: 26.09.2013 DE 102013219432
(43) Veröffentlichungstag der Anmeldung: 25.05.2016
(73) Patentinhaber: Röhr, Peter, 38128 Braunschweig (DE); Vetter, Joachim, 38259 Salzgitter (DE); Schierwater, Bernd, 38128 Braunschweig (DE)
(72) Erfinder: Röhr, Peter, 38128 Braunschweig (DE); Vetter, Joachim, 38259 Salzgitter (DE); Schierwater, Bernd, 38128 Braunschweig (DE)
(74) Vertreter: Plöger, Jan Manfred
(86) Internationale Anmeldenummer: PCT/EP2014/002615
(87) Internationale Veröffentlichungsnummer: WO 2015/043754

(56) Entgegenhaltungen:
- WO-A1-02/091922
- WO-A1-03/030984
- DE-T2- 60 225 859
- US-A1- 2012 271 125
- US-A1- 2013 018 279

## Beschreibung

Die Erfindung betrifft eine Blutentnahmevorrichtung gemäß dem Oberbegriff von Anspruch 1.

In einer Vielzahl von Situationen ist es notwendig, von einem Patienten, bei dem es sich um einen menschlichen Patienten oder ein Tier handeln kann, Blut zu entnehmen. In aller Regel wird dazu entweder eine Vene angestochen und Blut entnommen, oder es wird mittels einer Nadel eine Wunde, beispielsweise in eine Fingerkuppe eingebracht, sodass aus Kapillaren Blut nach außen austritt, das dann aufgefangen wird. Dieses Verfahren wird beispielsweise von Diabetikern angewendet, die in regelmäßigen Abständen ihren Blutzuckerspiegel erfassen müssen.

Nachteilig an der Blutentnahme aus Venen ist, dass sie schmerzhaft ist und zudem nur von medizinisch geschultem Personal durchgeführt werden kann, um größere Verletzungen zu vermeiden. Nachteilig am Anstechen beispielsweise der Fingerkuppen ist, dass nur eine geringe Menge Blut erhalten wird, das zudem durch Kontakt mit der Hautoberfläche verunreinigt sein kann. Nachteilig ist zudem, dass viele Menschen davor zurückschrecken, sich selbst eine blutende Wunde zuzufügen.

Eine gattungsgemäße Blutentnahmevorrichtung ist aus der DE 602 25 859 T2 bekannt. Nachteilig an dem dort beschriebenen System ist, dass das Entnehmen besonders größerer Mengen an Blut schmerzhaft ist.

Aus der WO 02/091 922 A1 ist ebenfalls eine gattungsgemäße Blutentnahmevorrichtung bekannt, die eine Membranpumpe zum Absaugen von Blut aus der Haut aufweist. Auch an dieser Blutentnahmevorrichtung ist nachteilig, dass sie zu Schmerzen führt.

In der US 2012/0271125 A1 ist eine Vorrichtung zum Abgeben von Medikamenten in die Haut und/oder zum Entnehmen von Flüssigkeiten aus der Haut bekannt. Durch eine besonders hohe Beschleunigung des Mikronadelfelds wird das Schmerzempfinden bei der Blutabnahme vermindert. Allerdings lässt sich das Schmerzempfinden so nicht vollständig ausschalten.

Der Erfindung liegt die Aufgabe zu Grunde, das Abnehmen von Blut schmerzärmer zu gestalten.

Die Erfindung löst das Problem durch eine gattungsgemäße Blutentnahmevorrichtung mit den Merkmalen von Anspruch 1.

Vorteilhaft an der Erfindung ist, dass durch die Verwendung des Mikronadelfelds mit einer hohen Wahrscheinlichkeit zumindest eine Mikronadel, insbesondere aber mehrere Mikronadeln, eine Kapillare in der Lederhaut oder ein Blutgefäß in der Unterhaut trifft. Aus diesem Grund können die Mikronadeln mit einem kleinen Durchmesser ausgeführt werden, sodass das Durchstechen der Unterhaut mit den Mikronadeln nur wenig Schmerzen bereitet.

Da ein Schmerzmittel in der Wirkflüssigkeit vorhanden ist, kann das Mikronadelfeld so in die Haut eingebracht werden, dass zunächst die Mikronadeln lediglich die schmerzunempfindliche Epidermis durchdringen. Durch das Abgeben des Schmerzmittels kann dann das Schmerzempfinden auch in den darunter liegenden Hautschichten ausgeschaltet werden. Es ist dann gemäß einer bevorzugten Ausführungsform vorgesehen, so lange zu warten, bis das Schmerzmittel das Schmerzempfinden zumindest weitgehend ausgeschaltet hat, und danach die Mikronadeln so tief in die Haut einzustoßen, dass zumindest eine Blut führende Kapillare von zumindest einer Mikronadel getroffen wird. Mittels der Unterdruckerzeugungsvorrichtung kann dann das Blut aus der Kapillare abgesaugt werden. Wird also Schmerzmittel verwendet, ist eine zumindest quasi schmerzfreie Blutentnahme möglich.

Die Dicke der schmerzunempfindlichen Epidermis variiert je nach Alter, Geschlecht und Körperregion. Es muss daher für jede Person eine geeignete Blutentnahmevorrichtung gewählt werden. Erfindungsgemäß ist daher auch ein Kit, das zumindest zwei, insbesondere aber mehrere, Blutentnahmevorrichtungen umfasst, wobei die Eintauchtiefe, also diejenige Länge, um die die Mikronadeln maximal über die Anlagefläche überstehen, der Blutentnahmevorrichtung mit der größten Eintauchtiefe zumindest 10%, insbesondere zumindest 20%, größer ist als die Eintauchtiefe der Blutentnahmevorrichtung mit der kleinsten Eintauchtiefe.

Da die Mikronadeln einen sehr geringen Durchmesser haben, ist es in bestimmten Einsätzen denkbar, dass kein Schmerzmittel verwendet wird. Das kann beispielsweise dann vorteilhaft sein, wenn das Blut an einer wenig schmerzempfindlichen Stelle abgenommen werden soll, beispielsweise am Oberschenkel oder am Rücken.

Zusätzlich zum Schmerzmittel ist ein Thrombozytenaggregationshemmer in der Wirkflüssigkeit vorhanden. Dieser verhindert die Gerinnung des Blutes, sodass solange gewartet werden kann, bis hinreichend viel Blut entnommen wurde, bevor die Blutentnahmevorrichtung vom Patienten abgenommen werden muss. Es ist allerdings in bestimmten Einsätzen denkbar, dass ohne einen Thrombozytenaggregationshemmer gearbeitet wird. Das gilt beispielsweise dann, wenn das Blut unmittelbar nach Entnahme aus dem Körper in Kontakt mit einem Mikrochip gebracht wird, der die gewünschte Analyse durchführt, bevor das Blut durch Gerinnung unanalysierbar wird.

Besonders günstig ist es, dass sowohl ein Thrombozytenaggregationshemmer als auch ein Schmerzmittel in der Wirkflüssigkeit vorhanden sind. In diesem Fall besteht aufgrund des Vorhandenseins des Thrombozytenaggregationshemmers kein Zeitdruck für die Entnahme des Blutes, sodass solange gewartet werden kann, bis das Schmerzmittel das Schmerzempfinden zumindest überwiegend ausgeschaltet hat. Das ermöglicht es, Blut in hinreichender Menge schmerzarm oder sogar schmerzfrei abzunehmen.

Im Rahmen der vorliegenden Beschreibung wird unter einer Blutentnahmevorrichtung eine Vorrichtung verstanden, die dazu ausgebildet ist, Blut zu entnehmen. Insbesondere sind die Unterdruckerzeugungsvorrichtung und das Mikronadelfeld zumindest mittelbar miteinander verbunden, sodass die Blutentnahmevorrichtung mit einer Hand gehalten und appliziert werden kann.

Unter der Wirkflüssigkeit wird insbesondere eine Flüssigkeit verstanden, die in der Blutentnahmevorrichtung vorhanden ist, bevor mit der Blutentnahmevorrichtung Blut von einem Patienten entnommen wird. Die Wirkflüssigkeit ist Teil der Blutentnahmevorrichtung.

Unter einem Schmerzmittel wird eine chemische Substanz verstanden, die temporär das Schmerzempfinden reduziert, insbesondere ausschaltet. Beispielsweise kommt als Schmerzmittel Mepivacain oder Meivacain in Betracht. Es kann sich bei dem Schmerzmittel um ein Lokalanästhetikum, beispielsweise ein Aminoamid oder einen Aminoester, oder ein Analgetikum handeln.

Unter einem Mikronadelfeld wird insbesondere eine Anordnung aus Mikronadeln verstanden, die örtlich so dicht zueinander angeordnet sind, dass sie, beispielsweise durch Bewegen der Blutentnahmevorrichtung mit einer Hand, gemeinsam in die Haut eines Patienten einbringbar sind. Beispielsweise ist das Mikronadelfeld so ausgebildet, dass es von einem gedachten Kreis mit einem Durchmesser von 10 cm, insbesondere von 5 cm, bevorzugt aber von 3 cm, umschlossen werden kann. Vorzugsweise umfasst das Mikronadelfeld zumindest 10 Mikronadeln, insbesondere zumindest 25 Mikronadeln. Auf diese Weise ist sichergestellt, dass mit hoher Wahrscheinlichkeit zumindest eine Mikronadel eine Kapillare trifft, aus der Blut entnommen werden kann.

Unter einer Mikronadel wird eine Nadel verstanden, deren Außendurchmesser höchstens 200 µm, insbesondere höchstens 100 µm, beträgt. Die Mikronadeln sind aus einem Material gefertigt, das hinreichend zäh ist, so dass ein Abbrechen vermieden wird.

Unter der Unterdruckerzeugungsvorrichtung wird insbesondere eine Vorrichtung verstanden, die nach Aktivieren selbsttätig einen Unterdruck erzeugt und/oder an das Mikronadelfeld anlegt. Die Unterdruckerzeugungsvorrichtung ist in anderen Worten insbesondere so ausgebildet, dass sie nach Aktivieren ohne Zutun von außen den Unterdruck aufrechterhält. Vorzugsweise erfolgt dies durch mechanische Mittel, insbesondere umfasst die Blutentnahmevorrichtung nur passive Bauteile. Ein passives Bauteil ist ein Bauteil, das nur gespeicherte mechanische Energie umsetzt, nicht aber chemische oder elektrische Energie. Vorzugsweise umfasst die Unterdruckerzeugungsvorrichtung also keine rotierenden Bauteile.

Vorzugsweise ist die Wirkflüssigkeit in einem Behälter angeordnet, der luftfrei ist. Es ist dann möglich, die Wirkflüssigkeit durch Ausüben eines Drucks auf diesen Behälter aus dem Behälter und die Mikronadeln in die Haut des Patienten abzugeben, ohne das befürchtet werden muss, das Luft in den Körper des Patienten gerät. Selbstverständlich ist es durchaus möglich, dass geringe Mengen an Luft vorhanden sind. Maßgeblich ist, dass diese Luftmengen so gering sind, dass sie keine Gefahr für den Patienten darstellen und die Entnahme des Blutes nicht gefährden.

Die Blutentnahmevorrichtung umfasst einen Blutbehälter, der mit einer Vielzahl der Mikronadeln verbunden ist, sodass Blut mittels der Mikronadeln in den Blutbehälter saugbar ist. Es ist möglich, nicht aber notwendig, dass alle Mikronadeln des Mikronadelfelds mit dem Blutbehälter verbunden sind. Es ist auch denkbar, dass die Mikronadeln mit einem Wirkflüssigkeitsbehälter verbunden sind, in dem die Wirkflüssigkeit angeordnet ist. Es ist dann möglich, die Wirkflüssigkeit durch die Mikronadeln in die Haut abzugeben.

Es ist zudem möglich, dass in diesem Fall zusätzlich zu den Mikronadeln Nadeln mit einem größeren Außendurchmesser vorhanden sind, die mit dem Blutbehälter in Verbindung stehen. In diesem Fall kann zunächst das Schmerzmittel durch die Mikronadeln in die Haut abgegeben werden. Sobald das Schmerzmittel wirkt, können die Nadeln mit dem größeren Durchmesser schmerzfrei in die Haut eingeführt werden. Eine derartige Blutentnahmevorrichtung hat den Vorteil, dass durch den größeren Durchmesser der Blutentnahmenadeln, also derjenigen Nadeln, durch die das Blut in den Blutbehälter saugbar ist, ein größerer Blutstrom fließen kann. Es kann auf diese Weise besonders viel Blut entnommen werden oder eine vorgegebene Menge Blut kann in kürzerer Zeit entnommen werden. Zwar muss dann in Kauf genommen werden, dass es nach der Blutentnahme und dem Unwirksamwerden des Schmerzmittels zu einem gegenüber der alleinigen Verwendung von Mikronadeln verstärkten Schmerzempfinden kommt, es kann jedoch sinnvoll sein, diesen Nachteil in Kauf zu nehmen, wenn die Vorteile, nämlich das mehr Blut oder in schnellerer Zeit entnommen werden kann, überwiegen.

Die Wirkflüssigkeit ist im Blutbehälter angeordnet. Dadurch ist es möglich, dass durch die Mikronadeln zunächst die Wirkflüssigkeit in die Haut des Patienten abgeben und dass danach durch die gleichen Mikronadeln Blut in den Blutbehälter eingesaugt wird. Eine derartige Blutentnahmevorrichtung ist konstruktiv besonders einfach aufgebaut. Günstig ist es zudem, wenn die Unterdruckerzeugungsvorrichtung mit dem Blutbehälter verbunden ist, sodass durch Erzeugen eines Überdrucks im Blutbehälter die Wirkflüssigkeit durch die Mikronadeln abgebbar und durch Erzeugen eines Unterdrucks im Blutbehälter Blut in den Blutbehälter saugbar ist.

Günstig ist es, wenn die Unterdruckerzeugungsvorrichtung eine Aktivierungsvorrichtung besitzt, mittels der die Unterdruckerzeugungsvorrichtung zum Erzeugen eines Unterdrucks aktivierbar ist. Es ist beispielsweise möglich, dass die Aktivierungsvorrichtung ausgebildet ist zum Zerstören einer Wand zwischen einem Vakuumbehälter, in dem ein Vakuum herrscht, und dem Blutbehälter. Alternativ oder zusätzlich ist es möglich, dass die Unterdruckerzeugungsvorrichtung eine vorgespannte Feder umfasst und dass die Aktivierungsvorrichtung die Feder in der vorgespannten Stellung hält, wobei durch zumindest teilweises Zerstören der Aktivierungsvorrichtung die Feder freigegeben wird und das Vakuum erzeugt.

Vorzugsweise besitzt die Blutentnahmevorrichtung eine Druckfläche, die so angeordnet ist, dass durch Drücken auf die Druckfläche die Wirkflüssigkeit unter Druck setzbar ist. Dadurch wird insbesondere bewirkt, dass die Wirkflüssigkeit durch die Mikronadeln abgegeben wird. Besonders günstig ist es, wenn die Druckfläche so ausgebildet ist, dass sie mit einem Finger oder mehreren Fingern betätigt werden kann.

Besonders günstig ist es, wenn die Aktivierungsvorrichtung so angeordnet ist, dass durch Druck auf die Druckfläche die Druckerzeugungsvorrichtung aktivierbar ist. Insbesondere ist die Aktivierungsvorrichtung durch Druck auf die Druckfläche, der in Richtung auf die Haut des Patienten zu verläuft, aktivierbar. In diesem Falle ist es ausreichend, Druck auf die Druckfläche auszuüben, um zunächst die Wirkflüssigkeit durch die Mikronadeln in die Haut des Patienten abzugeben und die Unterdruckerzeugungsvorrichtung zu aktivieren. Nach einem einmaligen Druck auf die Druckfläche kann die Blutentnahmevorrichtung dann auf der Haut des Patienten verbleiben, wo sie selbsttätig Blut entnimmt, ohne dass eingegriffen werden muss.

Gemäß einer bevorzugten Ausführungsform umfasst die Blutentnahmevorrichtung eine Befestigungsvorrichtung, mittels der die Blutentnahmevorrichtung an der Haut eines Patienten lösbar befestigbar ist. Beispielsweise umfasst die Befestigungsvorrichtung ein Klebeelement, mittels dem die Blutentnahmevorrichtung auf der Haut, insbesondere luftdicht, festklebbar ist. Unter dem Merkmal, dass die Blutentnahmevorrichtung auf der Haut luftdicht festklebbar ist, wird insbesondere verstanden, dass so wenig Luft durchgelassen wird, dass die Blutentnahmevorrichtung mittels eines Unterdrucks auf der Haut haltbar ist. Auf diese Weise kann das Klebeelement mit einem vergleichsweise schwachen Kleber und mit einer vergleichsweise kleinen Klebefläche ausgebildet werden, da der Unterdruck die Befestigung auf der Haut unterstützt. Es ist aber auch möglich, dass die Blutentnahmevorrichtung auch ohne Anlegen eines Unterdrucks mittels der Befestigungsvorrichtung an der Haut des Patienten haltbar ist.

Günstig ist es, wenn das Klebeelement einen Schaum umfasst, wobei dieser Schaum vorzugsweise nicht rückfedernd ist. Hierunter wird insbesondere verstanden, dass er dann, wenn er zusammengedrückt wird, innerhalb von 30 Sekunden auch ohne ständiges Anlegen einer Kraft höchstens 70% seiner Ausgangsdicke einnimmt. Auf diese Weise können die Mikronadeln in die Haut eingebracht werden, ohne das eine große Kraft aufgebracht werden müsste, um die Blutentnahmevorrichtung am Platze zu halten.

Es ist möglich, dass der Schaum Klebstoff enthält, der in Poren des Schaums angeordnet ist. Günstig ist es, wenn das Klebeelement mit einer entfernbaren Schutzabdeckung abgedeckt ist.

Vorzugsweise besitzt die Blutentnahmevorrichtung eine Anlagefläche zum Anlegen der Blutentnahmevorrichtung an die Haut eines Patienten und ein Kompressionselement, dass durch Druck aus einem unkomprimierten Zustand in einen komprimierten Zustand bringbar ist, wobei die Mikronadeln zumindest dann, wenn das Kompressionselement im komprimierten Zustand ist, und zumindest 0,7 mm, insbesondere 1,0 mm, über die Anlagefläche überstehen. Es ist günstig, wenn die Mikronadeln dann, wenn das Kompressionselement im unkomprimierten Zustand ist, nicht über die Anlagefläche überstehen. In diesem Fall sind die Mikronadeln nicht oder kaum sichtbar, wenn die Blutentnahmevorrichtung appliziert wird. Das Kompressionselement kann gemäß einer bevorzugten Ausführungsform durch Schaumstoff gebildet sein, insbesondere auch durch den Schaumstoff des Klebeelements.

Wenn die Mikronadeln zumindest 0,7 Millimeter über das Kompressionselement überstehen, wenn also die Eintauchtiefe zumindest 0,7 Millimeter beträgt, kann bei einem Säugling oder einer Sechzigjährigen aus den Blutgefäßen der Unterhaut an verschiedenen Teilen des Körpers, beispielsweise dem Handballen oder der Fingerkuppe, Blut entnommen werden. Bei einem körperlich arbeitenden Mann hingegen sollte die Eintauchtiefe größer als 0,7 Millimeter sein, beispielsweise 1 Millimeter. Es kann dann aber an vielen Stellen des Körpers dieses Mannes Blut entnommen werden.

Unter der Anlagefläche wird diejenige Fläche verstanden, mit der die Blutentnahmevorrichtung an der Haut des Patienten anliegt, wenn kein Druck auf die Blutentnahmevorrichtung ausgeübt wird. Beispielsweise ist die Anlagefläche die Oberfläche des Klebeelements.

Alternativ oder zusätzlich kann die Befestigungsvorrichtung eine Manschette umfassen, mittels der die Blutentnahmevorrichtung formschlüssig an einem Patienten befestigbar ist. Insbesondere handelt es sich bei der Manschette um eine aufblasbare Manschette, sodass mittels der Manschette auch der Blutdruck gemessen werden kann.

Günstig ist es, wenn die Mikronadeln so lang ausgebildet sind, dass sie dann, wenn die Blutentnahmevorrichtung angeordnet ist und Druck auf die Druckfläche ausgeübt wird, um zumindest 1,5 mm, insbesondere zumindest 2 mm, in die Haut einbringbar sind. Die Lederhaut hat eine Dicke von ca. 1,5 mm, sodass bei einem tieferen Einbringen als 1,5 mm mit hoher Wahrscheinlichkeit eine blutführende Kapillare oder ein Blutgefäß getroffen wird.

Gemäß einer bevorzugten Ausführungsform umgrenzt das Klebeelement einen Saugbereich und die Blutentnahmevorrichtung weist eine Unterdruckkammer auf, die zum Saugbereich geöffnet ist, sodass die Blutentnahmevorrichtung mittels des in der Unterdruckkammer vorhandenen Unterdrucks an der Haut des Patienten festsaugbar ist. Die Unterdruckkammer kann beispielsweise eine Feder aufweisen, die von einer Aktivierungsvorrichtung in einer vorgespannten Position gehalten wird, wobei diese Aktivierungsvorrichtung die vorgespannte Feder freigibt, wenn Druck auf die Druckfläche ausgeübt wird. Es ist allerdings auch möglich, dass die Aktivierungsvorrichtung auf eine andere Art und Weise aktiviert wird. Diese Unterdruckkammer ist entbehrlich, wenn die Befestigungsvorrichtung eine Manschette umfasst, da die Blutentnahmevorrichtung dann mittels der Manschette relativ zum Körper des Patienten fixierbar ist.

Günstig ist es, wenn das Mikronadelfeld vom Wirkstoffbehälter lösbar ausgebildet ist. In diesem Fall ist es möglich, nach erfolgter Blutentnahme das Mikronadelfeld zu entfernen, sodass eine Gefährdung ausgeschlossen wird. Vorzugsweise ist das Mikronadelfeld ohne Werkzeug und insbesondere ohne Zerschneiden lösbar, also beispielsweise verrastet, verklebt oder per Perforation mit dem Wirkstoffbehälter verbunden. Es ist möglich, nicht aber notwendig, dass das Mikronadelfeld unmittelbar mit dem Wirkstoffbehälter verbunden ist, es ist auch möglich, dass eine mittelbare Verbindung besteht.

Gemäß einer bevorzugten Ausführungsform ist im Blutbehälter ein Mikrochip angeordnet, mittels dem zumindest eine Eigenschaft des Bluts quantitativ und/oder qualitativ ermittelbar ist. Insbesondere kann der Mikrochip ausgebildet sein zum Durchführen zumindest eines Gentests.

Vorzugsweise umfasst der Mikrochip eine Energiewandeleinheit, mittels der eingestrahlte Energie in elektrische Energie umgewandelt werden kann. Beispielweise umfasst die Energiewandeleinheit eine Spule, sodass durch Anlegen eines elektrischen externen Wechselfelds ein elektrischer Strom im Mikrochip induzierbar ist. Möglich ist auch, dass der Mikrochip durch Bestrahlung mit Licht auslesbar ist.

Vorzugsweise umfasst die Befestigungsvorrichtung eine expandierbare, insbesondere aufblasbare Manschette, an der die Blutentnahmevorrichtung befestigt sein kann, wobei die Blutentnahmevorrichtung so relativ zur aufblasbaren Manschette angeordnet ist, dass durch Expandieren der Manschette so Druck auf die Blutentnahmevorrichtung ausübbar ist, dass die Wirkflüssigkeit durch das Mikronadelfeld abgebbar ist. In anderen Worten kann durch Aufblasen der Manschette der Wirkstoff in die Haut des Patienten abgegeben werden. Beispielsweise durch Verringern des Drucks in der Manschette kann dann in einem nachfolgenden Schritt das Blut von der Unterdruckerzeugungsvorrichtung in den Blutbehälter gesaugt werden.

Gemäß einer bevorzugten Ausführungsform umfasst die Blutentnahmevorrichtung eine Druckerzeugungseinheit, die mit der Manschette zum Anlegen eines Überdrucks verbunden ist, und eine Ansteuereinheit, die ausgebildet ist zum automatischen Durchführen eines Verfahrens mit den Schritten (i) Ansteuern der Druckerzeugungseinheit, sodass diese die Manschette mit einem Wirkstoffabgabe-Druck beaufschlagt, wobei der Wirkstoffabgabe-Druck so gewählt ist, dass die Wirkflüssigkeit durch die Mikronadeln abgegeben wird, und danach Ansteuern der Druckerzeugungseinheit, sodass diese die Manschette mit einem Entnahme-Druck beaufschlagt, wobei der Entnahme-Druck so gewählt ist, dass Blut mittels der Mikronadeln ansaugbar ist. In diesem Fall muss lediglich die Blutentnahmevorrichtung korrekt am Körper des Patienten, beispielsweise an einer Gliedmaße, befestigt werden. Die Ansteuereinheit führt danach automatisch, das heißt selbsttätig, alle Schritte aus, die notwendig sind, um Blut vom Patienten zu entnehmen. Nach der Blutentnahme muss lediglich die Manschette abgenommen werden und die Blutprobe ist entnommen. Medizinisches Fachpersonal ist entbehrlich.

Besonders bevorzugt ist die Ansteuereinheit ausgebildet zum zusätzlichen Durchführen des Schritts eines Ansteuerns der Druckerzeugungseinheit, sodass diese an die Manschette einen Start-Druck anlegt, wobei der Start-Druck so gewählt ist, dass das Kompressionselement in den komprimierten Zustand gebracht wird. Das ist dann besonders vorteilhaft, wenn die Mikronadeln nicht über das Kompressionselement im unkomprimierten Zustand hinausragen. In anderen Worten kann die Blutentnahmevorrichtung am Patienten platziert werden, ohne dass dieser die Nadeln sieht oder spüren kann. Durch das Anlegen der oben genannten unterschiedlichen Drücke kann dann auf schmerzarme oder schmerzfreie Weise Blut entnommen werden.

Gemäß einer bevorzugten Ausführungsform enthält die Wirkflüssigkeit zumindest eine gefäßerweiternde Substanz, beispielsweise einen Beta-Blocker oder einen Calciumantagonisten.

Gemäß einer bevorzugten Ausführungsform enthält die Wirkflüssigkeit zumindest ein Antikoagulantium. Unter einem Antikoagulantium wird eine Substanz verstanden, die die Blutgerinnung hemmt. Das Antikoagulantium kann ein direktes oder ein indirektes Antikoagulantium sein. Beispiele für Antikoagulantia sind Hirudin, Apixaban, Dabigatran, Rivaroxaban sowie Vitamin-K-Antagonisten und Heparine.

Insbesondere enthält die Wirkflüssigkeit also ein Antikoagulantium, einen Thrombozytenaggregationshemmer, einen Vasodilatator und ein Lokalanästhetikum oder Analgetikum.

Im Folgenden wird die Erfindung anhand der beigefügten Zeichnungen näher erläutert. Dabei zeigt
- Figur 1: eine schematische Querschnittsansicht einer erfindungsgemäßen Blutentnahmevorrichtung,
- Figur 2: eine Ansicht von unten auf die Blutentnahmevorrichtung gemäß Figur 1,
- Figur 3: die Blutentnahmevorrichtung nach dem Abgeben der Wirkflüssigkeit,
- Figur 4: die Blutentnahmevorrichtung beim Entnehmen von Blut und
- Figur 5: die Blutentnahmevorrichtung nach der Blutentnahme.
- Figur 6: zeigt in seinen Teilfiguren 6a bis 6f schematisch das Entnehmen von Blut mittels der Blutentnahmevorrichtung.
- Figur 7: zeigt eine schematische Querschnittsansicht einer erfindungsgemäßen Blutentnahmevorrichtung gemäß einer zweiten Ausführungsform nach dem Einsaugen von Blut und
- Figur 8: die Blutentnahmevorrichtung gemäß Figur 7 mit abgenommenem Mikronadelfeld.

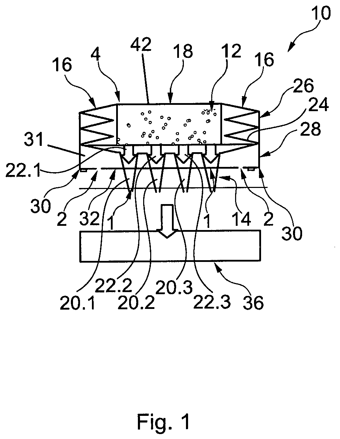

Figur 1 zeigt eine erfindungsgemäße Blutentnahmevorrichtung 10, die eine Wirkflüssigkeit 12, ein Mikronadelfeld 14 und eine Unterdruckerzeugungsvorrichtung 16 umfasst. Die Wirkflüssigkeit 12 hat ein Volumen V₁₂ von beispielsweise weniger als 75 Mikroliter, insbesondere weniger als 50 Mikroliter. Im vorliegenden Fall sind 25 Mikroliter Wirkflüssigkeit vorhanden. Die Wirkflüssigkeit enthält einen Thrombozytenaggregationshemmer, beispielsweise in Form von Heparin, und/oder ein Schmerzmittel, beispielsweise 1% Mepivacain bzw. Meavirin. Es ist jedoch auch möglich, andere Lokalanästhetika zu verwenden.

Die Wirkflüssigkeit ist in einem Behälter 18 angeordnet, der im vorliegenden Fall zudem als Blutbehälter fungiert. Es ist jedoch auch möglich, dass die Wirkflüssigkeit 12 in einem gesonderten Wirkstoffbehälter, der nicht gleichzeitig der Blutbehälter ist, untergebracht ist.

Das Mikronadelfeld 14 umfasst eine Vielzahl an Mikronadeln 20.1, 20.2,..., die im vorliegenden Fall alle mit dem Blutbehälter 18 über Kanäle 22.1, 22.2,... in Verbindung stehen. Insgesamt sind in der vorliegenden Ausführungsform 25 Mikronadeln vorhanden, es ist aber auch möglich, dass mehr oder weniger Mikronadeln verwendet werden.

Die Unterdruckerzeugungsvorrichtung 16 umfasst zumindest eine Feder 24, die beispielsweise aus Kunststoff oder aus Metall gefertigt sein kann. Die Feder 24 wird durch eine Aktivierungsvorrichtung 26 im vorgespannten Zustand gehalten, sodass sie sich nicht längen kann.

Die Blutentnahmevorrichtung 10 besitzt eine Befestigungsvorrichtung 28, die in der gezeigten Ausführungsform ein Klebeelement 30 umfasst. Das Klebeelement ist vorliegend durch einen mit Klebstoff zumindest teilweise getränktes Kompressionselement 31 in Form eines Schaumkörpers gebildet. Das Kompressionselement 31 ist zumindest auch um das Mikronadelfeld 14 angeordnet ist.

An der Befestigungsvorrichtung 28 ist eine Anlagefläche 32 ausgebildet, die im vorliegenden Fall auf der dem Behälter 18 abgewandten Seite angeordnet ist. Mit der Anlagefläche 32 wird die Blutentnahmevorrichtung 10 im Einsatzfall mit einer Haut 34 eines Patienten in Kontakt gebracht. Figur 1 zeigt zudem eine Schutzabdeckung 36, die so ausgebildet ist, dass das Mikronadelfeld 14 nicht freiliegt, wenn die Schutzabdeckung 36 aufgesetzt ist.

Figur 1 zeigt zudem, dass die Blutentnahmevorrichtung 10 eine Druckfläche 42 besitzt, die in der vorliegenden Ausführungsform am Behälter 18 ausgebildet ist.

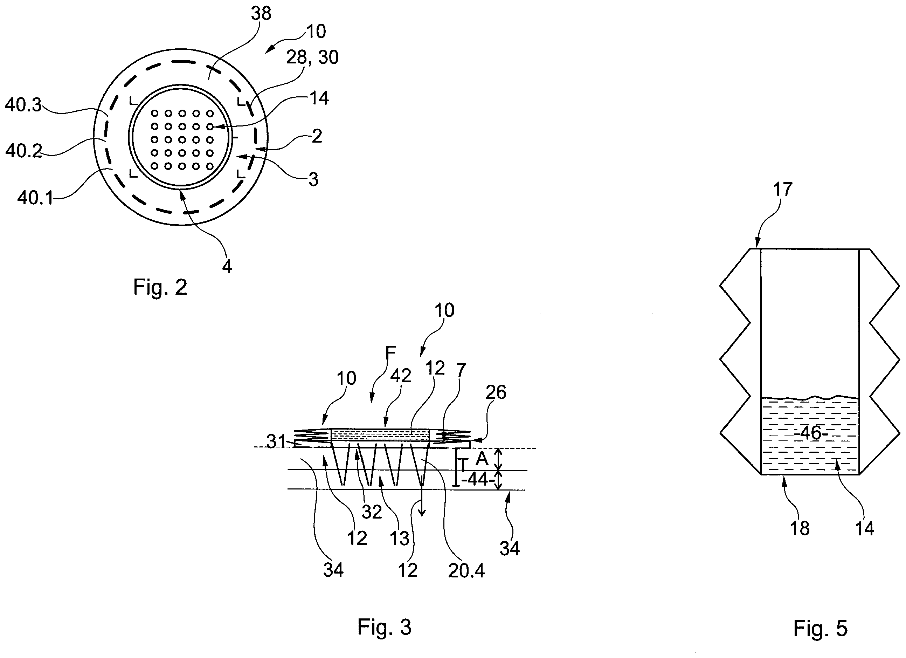

Figur 2 zeigt eine Ansicht von unten auf die Blutentnahmevorrichtung 10. Es ist zu erkennen, dass das Klebeelement 30 ringförmig ausgebildet ist. Das Klebeelement 30 umschließt einen Saugraum 38, in dem sich bei Anwendung der Blutentnahmevorrichtung ein Unterdruck ausbilden kann, wie weiter unten beschrieben wird. Es ist möglich, dass das Klebeelement 30, wie in Figur 2 gezeigt, Unterbrechungen 40.1, 40.2,... aufweist, sodass Luft mit einem geringen Volumenstrom in den Saugraum 38 einströmen kann.

Figur 2 zeigt zudem, dass eine kleine Menge an Wirkflüssigkeit im Behälter 18 verblieben ist. In den Blutbehälter 18 in späteren Schritten (wie im Folgenden beschrieben) einströmendes Blut wird durch das in der Wirkflüssigkeit 12 enthaltene Heparin am Gerinnen gehindert.

Figur 3 zeigt den Zustand, nachdem eine Kraft F auf die Druckfläche 42 ausgeübt wurde, sodass die Wirkflüssigkeit 12 durch die Mikronadeln 20 (Bezugszeichen ohne Zählsuffix beziehen sich auf alle entsprechenden Elemente) in eine Unterhaut 44 der Haut 34 abgegeben wurde. Beim Ausüben der Kraft F auf die Druckfläche 42 wurde die Aktivierungsvorrichtung 26 dadurch aktiviert, dass sie gebrochen ist. Dazu kann die Aktivierungsvorrichtung 26 beispielsweise eine nicht eingezeichnete Sollbruchstelle aufweisen.

Figur 3 illustriert zudem, dass die Mikronadeln 20 des Mikronadelfelds 14 mit einer Eintauchtiefe T über die Anlagefläche 32 überstehen, wenn eine Kraft F auf die Druckfläche 42 ausgeübt wird, die so groß ist, dass Wirkflüssigkeit 12 in zum Anästhesieren relevanter Menge abgegeben wird. Die Eintauchtiefe liegt zwischen 0,6 und 1,5 Millimetern und hängt von der Nachgiebigkeit des Kompressionselements 31 ab.

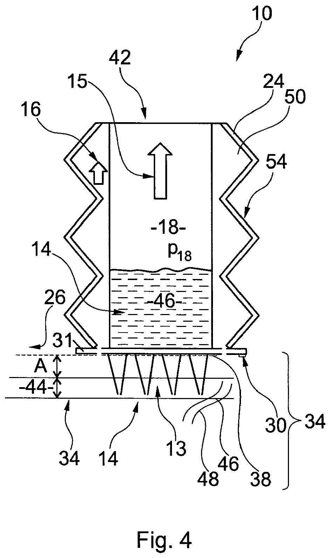

Figur 4 zeigt den Zustand, in dem die Feder 24 aus ihrer vorgespannten Lage in ihre ausgedehnte Lage zurückfedert. Dabei entsteht im Blutbehälter 18 ein Unterdruck p₁₈. Durch diesen Unterdruck wird Blut 46 aus einer schematisch eingezeichneten Kapillaren 48 in den Blutbehälter 18 gesaugt.

Durch das Expandieren der Feder 24, bei der es sich beispielsweise um eine Spiralfeder handelt, entsteht zudem ein Unterdruck p₅₀ in einer Unterdruckkammer 50. Die Unterdruckkammer 50 ist über eine Öffnung 52 mit dem Saugraum 38 verbunden. Dadurch wird die Blutentnahmevorrichtung 10 auf die Haut 34 zu gesaugt und fest mit dieser verbunden. Es kann vorteilhaft sein, ein Ventil oder eine Düse 54 vorzusehen, sodass Luft in die Unterdruckkammer 50 einströmt und sicherstellt, dass sich die Druckfläche 42 weiter von der Haut 34 entfernt, sodass der Unterdruck p₁₈ im Blutbehälter 18 aufrecht erhalten wird.

Figur 5 zeigt den Zustand, in dem das Mikronadelfeld 14 (vergleiche Figur 4) entfernt wurde. Das Blut 46 ist nun im Blutbehälter 18 angeordnet und kann analysiert werden.

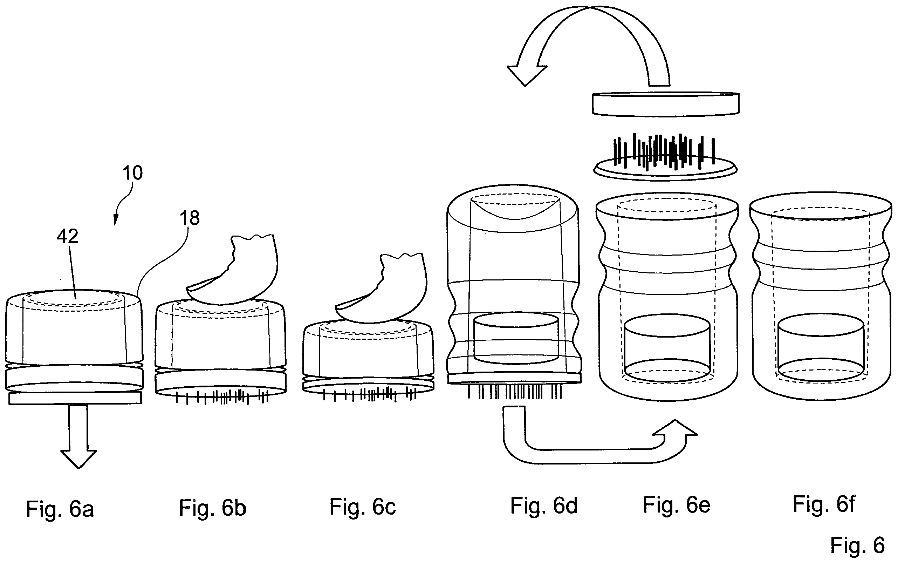

Figur 6 zeigt an den Teilfiguren 6a bis 6f in einem weiteren Beispiel die Funktionsweise der Blutentnahmevorrichtung 10. Es ist zu erkennen, dass der Blutbehälter 18 durch einen flexiblen Kunststoffbehälter gebildet ist.

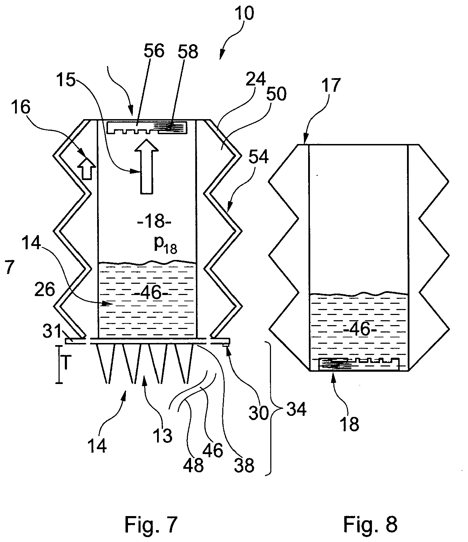

Figur 7 zeigt eine alternative Ausführungsform, die einen Mikrochip 56 umfasst. Dieser kann auf der dem Mikronadelfeld 14 abgewandten Seite des Blutbehälters 18 angeordnet sein. Wie Figur 8 zeigt, kann das Blut durch Umdrehen des Blutbehälters 18 in Kontakt mit dem Mikrochip 56 gebracht werden. Durch eine Induktionsspule 58 wird der Mikrochip 56 durch Anlegen eines äußeren Wechselfelds mit Strom versorgt.

**Bezugszeichenliste**

| | | | |
|---|---|---|---|
| 10 | Blutentnahmevorrichtung | | |
| 12 | Wirkflüssigkeit | 50 | Unterdruckkammer |
| 14 | Mikronadelfeld | 52 | Öffnung |
| 16 | Unterdruckerzeugungsvorrichtung | 54 | Ventil |
| | | 56 | Mikrochip |
| 18 | Behälter, Blutbehälter | 58 | Induktionsspule |
| | | | |
| 20 | Mikronadel | F | Kraft |
| 22 | Kanal | p₁₈ | Druck |
| 24 | Feder | p₅₀ | Druck |
| 26 | Aktivierungsvorrichtung | T | Eintauchtiefe |
| 28 | Befestigungsvorrichtung | V₁₂ | Volumen |
| | | | |
| 30 | Klebeelement | | |
| 31 | Kompressionselement | | |
| 32 | Anlagefläche | | |
| 34 | Haut | | |
| 36 | Schutzabdeckung | | |
| 38 | Saugraum | | |
| | | | |
| 40 | Unterbrechung | | |
| 42 | Druckfläche | | |
| 44 | Unterhaut | | |
| 46 | Blut | | |
| 48 | Kapillare | | |

## Patentansprüche

1. Blutentnahmevorrichtung (10) mit
(a) einer Wirkflüssigkeit (12), die einen Thrombozytenaggregationshemmer enthält,
(b) einem Mikronadelfeld (14), das eine Vielzahl an Mikronadeln (20) aufweist,
(c) wobei eine Vielzahl an Mikronadeln (20) zum Abgeben der Wirkflüssigkeit (12) in die Haut (34) eines Patienten ausgebildet ist, und
(d) einer Unterdruckerzeugungsvorrichtung (16), die mit einer Vielzahl an Mikronadeln (20) verbunden ist, sodass Blut (46) mittels der Mikronadeln (20) aus der Haut (34) saugbar ist;
**dadurch gekennzeichnet, dass**
(e) die Wirkflüssigkeit (12) ein Schmerzmittel enthält,
(f) die Wirkflüssigkeit (12) im Blutbehälter (18) angeordnet ist und dass
(g) die Unterdruckerzeugungsvorrichtung (16) mit dem Blutbehälter (18) verbunden ist, sodass durch Erzeugen eines Überdrucks im Blutbehälter (18) die Wirkflüssigkeit (12) durch Mikronadeln (20) abgebbar und durch Erzeugen eines Unterdrucks Blut (46) in den Blutbehälter (18) saugbar ist.

2. Blutentnahmevorrichtung (10) nach einem der vorstehenden Ansprüche, **dadurch gekennzeichnet, dass**
die Unterdruckerzeugungsvorrichtung (16) eine Aktivierungsvorrichtung (26) besitzt, mittels der die Unterdruckerzeugungsvorrichtung (16) zum Erzeugen eines Unterdrucks aktivierbar ist.

3. Blutentnahmevorrichtung (10) nach einem der vorstehenden Ansprüche, **gekennzeichnet durch**
eine Druckfläche (42), die so angeordnet ist, dass durch Ausüben einer Kraft (F) auf die Druckfläche (42) die Wirkflüssigkeit (12) unter Druck (p₁₈) setzbar ist, wobei die Aktivierungsvorrichtung (26) so angeordnet ist, dass durch Ausüben einer Kraft (F) auf die Druckfläche (42) die Unterdruckerzeugungsvorrichtung (16) aktivierbar ist.

4. Blutentnahmevorrichtung (10) nach einem der vorstehenden Ansprüche, **gekennzeichnet durch** eine Befestigungsvorrichtung (28), mittels der die Blutentnahmevorrichtung (10) an der Haut (34) eines Patienten lösbar befestigbar ist.

5. Blutentnahmevorrichtung (10) nach einem der vorstehenden Ansprüche, **gekennzeichnet durch**
- eine Anlagefläche (32) zum Anlegen der Blutentnahmevorrichtung (10) an die Haut (34) eines Patienten und
ein Kompressionselement (31), das durch Druck (p₁₈) aus einem unkomprimierten Zustand in einen komprimierten Zustand bringbar ist,
- wobei die Mikronadeln (20) zumindest dann, wenn das Kompressionselement (31) im komprimierten Zustand ist, zumindest 1 Millimeter über die Anlagefläche (32) überstehen.

6. Blutentnahmevorrichtung (10) nach Anspruch 5, **dadurch gekennzeichnet, dass**
die Mikronadeln (20) dann, wenn das Kompressionselement (31) im unkomprimierten Zustand ist, nicht über die Anlagefläche (32) überstehen.

7. Blutentnahmevorrichtung (10) nach einem der Ansprüche 4 bis 6, **gekennzeichnet durch**
- eine Befestigungsvorrichtung (28), mittels der die Blutentnahmevorrichtung (10) an der Haut (34) eines Patienten lösbar befestigbar ist,
- wobei die Befestigungsvorrichtung (28) insbesondere eine Manschette umfasst, mittels der die Blutentnahmevorrichtung (10) formschlüssig an einem Patienten befestigbar ist.

8. Blutentnahmevorrichtung (10) nach einem der vorstehenden Ansprüche, **dadurch gekennzeichnet, dass** die Mikronadeln (20) so lang ausgebildet sind, dass sie dann, wenn die Blutentnahmevorrichtung (10) auf der Haut (34) eines Patienten angeordnet ist und Druck (p₁₈) auf die Druckfläche (42) ausgeübt wird, um zumindest 0,7 Millimeter in die Haut (34) eindringen.

9. Blutentnahmevorrichtung (10) nach einem der vorstehenden Ansprüche, **dadurch gekennzeichnet, dass**
- das Klebeelement (30) einen Saugbereich umgrenzt, und
- die Blutentnahmevorrichtung (10) eine Unterdruckkammer (50) aufweist, die zum Saugbereich geöffnet ist, so dass die Blutentnahmevorrichtung (10) mittels in der Unterdruckkammer (50) vorhandenen Unterdrucks an der Haut (34) des Patienten festsaugbar ist.

10. Blutentnahmevorrichtung (10) nach einem der vorstehenden Ansprüche, **dadurch gekennzeichnet, dass**
- die Befestigungsvorrichtung (28) eine expandierbare, insbesondere aufblasbare, Manschette umfasst, und
- die Blutentnahmevorrichtung (10) so relativ zur aufblasbaren Manschette anordenbar ist, dass durch Expandieren der Manschette so Druck (p₁₈) auf die Blutentnahmevorrichtung (10) ausübbar ist, dass die Wirkflüssigkeit (12) durch das Mikronadelfeld (14) abgebbar ist, wobei
die Blutentnahmevorrichtung (10)
(a) eine Druckerzeugungseinheit, die mit der Manschette zum Anlegen eines Überdrucks verbunden ist, und
(b) eine Ansteuereinheit aufweist, die ausgebildet ist zum automatischen Durchführen eines Verfahrens mit den Schritten:
(i) Ansteuern der Druckerzeugungseinheit, so dass diese die Manschette mit einem Wirkstoffabgabe-Druck beaufschlagt, wobei der Wirkstoffabgabe-Druck (p₁₈) so gewählt ist, dass die Wirkflüssigkeit (12) durch die Mikronadeln (20) abgegeben wird, und
(ii) Ansteuern der Druckerzeugungseinheit, sodass diese die Manschette mit einem Entnahme-Druck beaufschlagt, wobei der Entnahme-Druck so gewählt ist, dass Blut (46) mittels der Mikronadeln (20) ansaugbar ist.

11. Blutentnahmevorrichtung nach einem der vorstehenden Ansprüche, **dadurch gekennzeichnet, dass** die Wirkflüssigkeit (12) eine gefäßerweiternde Substanz enthält.

12. Blutentnahmevorrichtung nach einem der vorstehenden Ansprüche, **dadurch gekennzeichnet, dass** die Wirkflüssigkeit (12) zumindest ein Antikoagulantium enthält.

## Claims

1. A blood sample collection device (10) with
a) an active liquid (12) that contains a platelet aggregation inhibitor,
b) a microneedle array (14) that features a plurality of microneedles (20),
c) wherein a plurality of microneedles (20) is provided for injection of the active liquid (12) into the skin (34) of a patient, and
d) a negative pressure generating device (16) that is connected to a plurality of microneedles (20), so that blood (46) can be drawn out of the skin (34) via the microneedles (20);
**characterized by** that
e) the active liquid (12) contains a painkiller,
f) the active liquid (12) is located within the blood receptacle (18) and that
g) the pressure generating device (16) is connected with the blood receptacle (18) in a way that allows to inject the active liquid (12) through the microneedles (20) by generating a positive pressure in the blood receptacle (18) and to suck up blood (46) into the blood receptacle (18) by generating a negative pressure.

2. A blood sample collection device (10) according to one of the aforementioned claims, **characterized by** that
the negative pressure generating device (16) possesses an activation device (26) that can activate the negative pressure generating device (16) to generate a negative pressure.

3. A blood sample collection device (10) according to one of the aforementioned claims, **characterized by**
a button area (42) that is located so that the active liquid (12) can be put under pressure (p₁₈) by exerting a force (F) onto the button area (42), wherein the activation device (26) is located so that the negative pressure generating device (16) can be activated by exerting a force (F) onto the button area (42).

4. A blood sample collection device (10) according to one of the aforementioned claims, **characterized by** a mounting device (28) which allows the blood sample collection device (10) to be temporarily fixed to the skin (34) of a patient.

5. A blood sample collection device (10) according to one of the aforementioned claims, **characterized by**
- a contact surface (32) for placing the blood sample collection device (10) against the skin (34) of a patient and
a compression compound (31), that can be brought from an uncompressed state into a compressed state by application of pressure (p₁₈),
- where the microneedles (20) surpass the contact surface (32) by at least 1 millimeter, at least when the compression compound (31) is in compressed state.

6. A blood sample collection device (10) according to one of the aforementioned claims, **characterized by** that
the microneedles (20) do not surpass the contact surface (32) when the compression compound (31) is in uncompressed state.

7. A blood sample collection device (10) according to one of the claims 4 to 6, **characterized by**
- a mounting device (28) with which the blood sample collection device (10) can be temporarily fixed to the skin (34) of a patient,
- where the mounting device (28) in particular comprises a cuff with which the blood sample collection device (10) can be fixed to the patient's skin with form-locking.

8. A blood sample collection device (10) according to one of the aforementioned claims, **characterized in that** the microneedles (20) are formed with such a length that they penetrate the patient's skin (34) by at least 0.7 millimeters when the blood sample collection device (10) is collocated on the skin (34) of a patient and a pressure (p₁₈) is applied onto the pressure patch (42).

9. A blood sample collection device (10) according to one of the aforementioned claims, **characterized in that**
- the adherent compound (30) surrounds a suction area and that
- the blood sample collection device (10) contains a negative pressure chamber (50) that is opened towards the suction area, so that the blood sample collection device (10) can be attached to the patient's skin using the suction caused by the negative pressure in the negative pressure generating device (50).

10. A blood sample collection device (10) according to one of the aforementioned claims, **characterized by** that
- the mounting device (28) contains an expandable, especially inflatable cuff, and
- the blood sample collection device (10) can be located in such a way in relation to the inflatable cuff that a pressure (p₁₈) can be exerted onto the blood sample collection device (10) by expansion of the cuff, so that the active liquid (12) can be injected through the microneedle array (14), and in that
the blood sample collection device (10) contains
(a) a pressure generating device that is connected to a cuff for application of a positive pressure, and
(b) an electronic control unit designed to automatically carry out an operation including the following steps:
(i) actuating the pressure generating device, so that it charges the cuff with an active compound release pressure (p₁₈), selected to release the active liquid (12) through the microneedles (20), and
(ii) actuating the pressure generating device, so that it charges the cuff with an extraction pressure, selected to suck up blood (46) via the microneedles (20).

11. A blood sample collection device according to one of the aforementioned claims, **characterized by** that the active liquid (12) contains a vasodilatory compound

12. A blood sample collection device according to one of the aforementioned claims, **characterized by** that the active liquid (12) contains at least one anticoagulant.

## Revendications

1. Dispositif de prélèvement de sang (10), comportant
(a) un liquide actif (12) qui comprend un antiagrégant plaquettaire,
(b) un champ de microaiguilles (14) qui présente une multitude de microaiguilles (20),
(c) une multitude de microaiguilles (20) étant réalisées pour administrer le liquide actif (12) dans la peau (34) d'un patient, et
(d) un dispositif de génération d'une dépression (16) qui est relié à la multitude de microaiguilles (20), de sorte que le sang (46) peut être aspiré hors de la peau (34) au moyen des microaiguilles (20);
**caractérisé en ce que**
(e) le liquide actif (12) comprend un analgésique,
(f) le liquide actif (12) est disposé dans le réservoir de sang (18), et
(g) le dispositif de génération de dépression (16) est relié au réservoir de sang (18), de telle sorte que par génération d'une surpression dans le réservoir de sang (18), le liquide actif (12) peut être administré par des microaiguilles (20) et du sang (46) peut être aspiré dans le réservoir de sang (18) par génération d'une dépression.

2. Dispositif de prélèvement de sang (10) selon la revendication précédente, **caractérisé en ce que**
le dispositif de génération de dépression (16) possède un dispositif d'activation (26) au moyen duquel le dispositif de génération de dépression (16) peut être activé pour générer une dépression.

3. Dispositif de prélèvement de sang (10) selon l'une des revendications précédentes, **caractérisé par**
une surface de pression (42) qui est agencée de telle sorte que par application d'une force (F) sur la surface de pression (42), le liquide actif (12) peut être mis sous pression (p₁₈), le dispositif d'activation (26) étant agencé de telle sorte que par application d'une force (F) sur la surface de pression (42), le dispositif de génération de dépression (16) est activable.

4. Dispositif de prélèvement de sang (10) selon l'une des revendications précédentes, **caractérisé par** un dispositif de fixation (28) au moyen duquel le dispositif de prélèvement de sang (10) peut être fixé de façon amovible sur la peau (34) d'un patient.

5. Dispositif de prélèvement de sang (10) selon l'une des revendications précédentes, **caractérisé par**
- une surface d'application (32) pour appliquer le dispositif de prélèvement de sang (10) contre la peau (34) d'un patient, et
un élément de compression (31) qui peut être amené d'un état non comprimé vers un état comprimé par la pression (p₁₈),
- les microaiguilles (20) dépassant d'au moins 1 millimètre au-delà de la surface d'application (32) au moins lorsque l'élément de compression (31) est dans l'état comprimé.

6. Dispositif de prélèvement de sang (10) selon la revendication 5, **caractérisé en ce que**
les microaiguilles (20) ne dépassent pas au-delà de la surface d'application (32) lorsque l'élément de compression (31) est dans l'état non comprimé.

7. Dispositif de prélèvement de sang (10) selon l'une des revendications 4 à 6, **caractérisé par**
- un dispositif de fixation (28) au moyen duquel le dispositif de prélèvement de sang (10) peut être fixé de façon amovible sur la peau (34) d'un patient,
- le dispositif de fixation (28) comportant en particulier une manchette au moyen de laquelle le dispositif de prélèvement de sang (10) peut être fixé en coopération de formes sur un patient.

8. Dispositif de prélèvement de sang (10) selon l'une des revendications précédentes, **caractérisé en ce que** les microaiguilles (20) ont une longueur telle que lorsque le dispositif de prélèvement de sang (10) est disposé sur la peau (34) d'un patient et qu'une pression (p₁₈) est exercée sur la surface de pression (42), elles pénètrent d'au moins 0,7 millimètre dans la peau (34).

9. Dispositif de prélèvement de sang (10) selon l'une des revendications précédentes, **caractérisé en ce que**
- l'élément adhésif (30) délimite une zone d'aspiration et
- le dispositif de prélèvement de sang (10) comprend une chambre sous dépression (50) qui est ouverte vers la zone d'aspiration, de sorte que le dispositif de prélèvement de sang (10) peut être fixé par effet de ventouse sur la peau (34) du patient au moyen de la dépression qui existe dans la chambre sous dépression (50).

10. Dispositif de prélèvement de sang (10) selon l'une des revendications précédentes, **caractérisé en ce que**
- le dispositif de fixation (28) comprend une manchette expansible, en particulier gonflable, et
- le dispositif de prélèvement de sang (10) peut être agencé par rapport à la manchette gonflable de telle sorte que par expansion de la manchette une pression (p₁₈) peut être exercée sur le dispositif de prélèvement de sang (10) de telle sorte que le liquide actif (12) peut être administré par le champ de microaiguilles (14),
dans lequel
le dispositif de prélèvement de sang (10) comprend
(a) une unité de génération de pression qui est reliée à la manchette pour appliquer une surpression, et
(b) une unité de pilotage qui est réalisée pour la mise en oeuvre automatique d'un procédé comprenant les étapes consistant à :
(i) piloter l'unité de génération de pression de manière à solliciter la manchette par une pression d'administration de l'agent actif, la pression (p₁₈) d'administration d'agent actif étant choisie de telle sorte que le liquide actif (12) est administré par les microaiguilles (20), et
(ii) piloter l'unité de génération de pression de manière à solliciter la manchette par une pression de prélèvement, la pression de prélèvement étant choisie de telle sorte que le sang (46) peut être aspiré au moyen des microaiguilles (20).

11. Dispositif de prélèvement de sang selon l'une des revendications précédentes, **caractérisé en ce que** le liquide actif (12) comprend une substance vasodilatatrice.

12. Dispositif de prélèvement de sang selon l'une des revendications précédentes, **caractérisé en ce que** le liquide actif (12) comprend au moins un anticoagulant.
